# EUROPEAN PATENT SPECIFICATION

(11) **EP 1 691 750 B1**
(45) Date of publication and mention of the grant of the patent: **27.04.2016**
(21) Application number: 04793843.6
(22) Date of filing: 25.10.2004
(51) Int. Cl.: A61F 13/56, A61F 13/84

(54) **DISPOSABLE ABSORBENT ARTICLE HAVING TENSION INDICATORS**
SAUGFÄHIGER EINWEGARTIKEL MIT SPANNUNGSINDIKATOREN
ARTICLE ABSORBANT JETABLE DOTE D'INDICATEURS DE TENSION

(30) Priority: 02.12.2003 SE 0303242
(43) Date of publication of application: 23.08.2006
(73) Proprietor: SCA Hygiene Products AB, 405 03 Göteborg (SE)
(72) Inventor: ZAJACZKOWSKI, Peter, Greenville SC 29601 (US)
(74) Representative: Zacco Sweden AB
(86) International application number: PCT/SE2004/001542
(87) International publication number: WO 2005/053588

(56) References cited:
- WO-A1-96/09027
- WO-A1-96/31175
- WO-A1-98/27921
- WO-A1-03/022730
- WO-A1-2005/037159
- GB-A- 1 332 148
- GB-A- 2 194 256
- US-A- 3 613 679
- US-A- 6 152 893
- US-B1- 6 432 074

## Description

### TECHNICAL FIELD

The present invention relates to a disposable absorbent article having a front part, a rear part and a crotch part extending between the front and rear part.

### BACKGROUND OF THE INVENTION

In order to obtain good fluid containment in a disposable absorbent article, such as an open type diaper, a pant diaper or an incontinence protector, a reasonably tight fit on the wearer is necessary. However, if the fit is overly tight, this can result in excess pressure on the skin of the wearer and lead to red marking or even skin abrasion. For persons with sensitive skin, like elderly persons, the consequences of an overly tight fit can be particularly severe because the skin may not regain its original shape or heal quickly. Articles of the type mentioned in the introduction are applied on the wearer by a caretaker or the wearer himself and the fit obtained is thus dependent on the person applying the article. There is thus a need for providing means for ensuring that the person applying the article will not tension the waistline of the article to such an extent that skin irritation will occur. There is also a need for indicating an appropriate tensioning in the leg elastics present in most articles of the above mentioned types. The fit of such articles is also dependent on that the right size for the wearer is chosen and it is therefore a need for means for indicating the tension in various parts of such articles, for example in the hip areas of pant diapers.

The objectives of the present invention is to prevent the person applying an article of the kind mentioned in the introduction from applying too much tension to the waistline of the article and also to indicate if the article applied is of an appropriate size for the wearer in question.

WO 2005/037159 published after the filing of the present priority application but having an earlier priority date discloses a diaper with a tension indicator of another type than the present invention. US 6,432,074 and US 3,613,679 discloses the use of tension indicators on elastic bandages.

### SUMMARY OF THE INVENTION

These objectives is achieved by a disposable absorbent article according to claim 1. By such an indicator it can be ensured that the article will have such a tight fit that good fluid containment can be obtained without risk for skin irritation, of course provided that the person applying the article take notice of the tension indication given. By the present invention it is also possible to indicate if a sufficiently tight fit is obtained.

The article can advantageously comprise elastic elements along leg openings and at least one tension indicator can be disposed adjacent to at least one of said leg openings in order to indicate if the leg elastics are overly tight or loose.

A tension indicator can be disposed visually on the outside of the article in at least one side part thereof and be placed in a region between a free end of said front or rear part and a leg opening. Such an embodiment is especially convenient for pant diapers in order to verify that a correct size of the article is used.

Advantageously, the article comprises tension indicators of two different types, wherewith one of said indicator types indicate that a minimum tension level is reached and the other that a maximum tension level is reached.

In the preferred embodiment, said strip of plastic material is attached to an outside of the article only in the opposite lateral ends of the strip and said strip of plastic material comprises a fold between its lateral ends.

In a further embodiment at least one portion of the rear or front part of the article comprises an outer sheet of a material which visual appearance changes when a certain tension is reached in the material.

### BRIEF DESCRIPTION OF THE DRAWINGS

The invention will now be described with reference to the enclosed Figures, of which;
Fig. 1 shows a schematic planar view of a disposable absorbent article according to a first preferred embodiment of the invention with the inside of the article turned against the viewer,
Fig. 2 shows a perspective view of the article of Figure 1 in an almost applied state with one pair of co-operating side portions of the front and rear parts fastened to each other,
Fig. 3 shows a sectional view along line III-III in Figure 1,
Fig. 4 shows a view similar to Figure 1 of an article according to a second preferred embodiment of the invention, and
Fig. 5 shows a sectional view along line V-V in Figure 4,
Fig. 6 shows a view similar to Figure 3 of an article according to a third preferred embodiment of the invention, and
Fig. 7 shows a view similar to Figure 3 of an article according to a fourth preferred embodiment of the invention.

### DESCRIPTION OF EMBODIMENTS

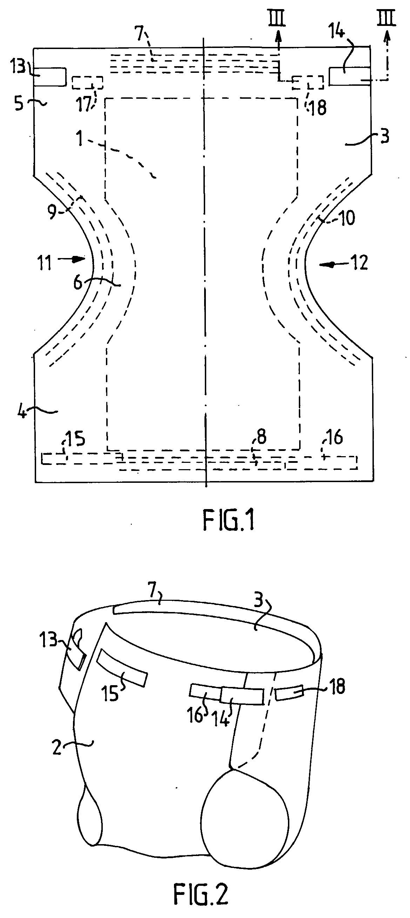

In Figures 1-3 a first preferred embodiment of an incontinence protector, i.e. a diaper for use by adults, is shown. As is conventional for such articles, said article comprises an absorbent body 1 enclosed between an outer liquid impermeable casing sheet 2 and an inner liquid permeable casing sheet 3. The article has an hour-glass shape with a front part 4, a rear part 5 and a crotch part 6 extending between the front and rear part. The casing sheets 2,3 are attached to each other in those portions that extend outside the absorbent body 1.

The article also comprises waist elastics in the form of several elastic threads 7,8 provided at the waistline of the front and rear parts, respectively, in the central parts thereof and attached to the casing sheets 2,3 in a pre-tensioned state. Pre-tensioned elastic threads 9,10 are also provided along the respective leg opening 11,12 of the article. Instead of elastic threads for the waist and/or the leg elastics it is of course possible to use elastic bands or bands of heat-shrinkable material, which shrink and obtain elastic properties after heat treatment. In Figure 1, the article is shown with the elastics in a pre-tensioned state, i.e. without the gathering of casing material that will occur when the elastic threads are allowed to relax. The elastic threads 7-10 are disposed between the casing sheets 2,3 but it is of course possible to dispose the elastic elements on the outside or the inside of the article, i.e. on the outer sheet 2 or the inner sheet 3.

Furthermore, the article comprises fasteners for giving the article a pants-like configuration when applied on a wearer, as is schematically shown in Figure 2. In the shown embodiment, the fasteners consist of tape tabs 13,14 co-operating with elongated strips 15,16 of a plastic material to which the tape tabs can be releasably attached and refastened several times, as is known in the art. Instead of tape tabs, mechanical fasteners, for example of the hook and loop type, can be used. It is to be noted that the fasteners should be of a type admitting adjustment of the length of the waistline so that a proper fit of the article can be obtained for wearers having different sizes and body shapes. In Figure 2, the article is shown with tape tab 14 fastened to strip 16. As can be understood, the amount of possible overlapping between the respective pairs of side portions of the front and rear parts is determined by the length of the strips 15,16. In the disclosed embodiment the tape tabs 13,14 are disposed on the side portions of the rear part and the strips of plastic material are disposed on the side portions of the front part. It is of course possible, but not preferred, to place the tape tabs on the side portions of the front part and the strips of plastic material on the side portions of the rear part.

The material in the outer casing sheet 2 may be polyethylene or any other materials known to be used as outer casing sheet in absorbent articles. Thus, the outer casing sheet can comprise several layers laminated to each other and may for aesthetic reasons have a fabric-like layer on its outside, i.e. the side distal from the wearer of the article. The outer casing layer is, as mentioned earlier, liquid impermeable but can advantageously be permeable for air and vapour.

The inner liquid permeable casing sheet 3 may be made of nonwoven material comprising fibres of polyethylene, polypropylene, polyester or mixtures thereof. Viscose fibres may also be used. It can also consist of a perforated plastic sheet. All types of materials known to be used as inner casing sheets in disposable absorbent can be used in an article according to the present invention.

The absorbent body 1 may contain cellulose fluff pulp with or without an admixture of particles and the like of so-called superabsorbent material and/or thermoplastic melt fibres. The absorbent body may also comprise several layers of absorbent material. Layers of nonwovens or the like for improving the liquid-acquisition properties of the absorbent body and layers for improving the liquid distribution may also be provided. All types of absorbent bodies known to be used in absorbent articles may be used in an article according to the invention.

In order to make use of the good fluid containment properties of today's disposable absorbent articles, such as diapers and incontinence protectors, it is essential that the article be properly fitted on the wearer. This means that when the article has been applied on a wearer, the waistline of the article will exert a pressure on the body of the wearer. If such pressure is too small, the article will probably leak, and if the pressure is to high, the skin of the wearer will be irritated or damaged. It has been found by the present inventor that the tension in the waistline should exceed 1 N in order to ensure a proper fluid containment and that if the tension in the waistline exceeds 8 N, damage to the skin is likely to be caused. However, the tension obtained in the waistline of the article is depending on the degree of overlapping of the side portions made by the person applying the article on a wearer. In order to help such a person to estimate how tight the article should be fitted on the wearer, the article according to the present invention is provided with tension indicators, such as the tension indicators 17,18 shown in Figures 1-3.

Tension indicators 17,18 consist of strips of plastic material which change its visual appearance when a certain tension is reached in the material. Examples of such plastic materials are unplasticized polyvinyl chloride, polyvinyl chloride-polyvinyl acetate copolymers, changing colours when subjected to cold flow deformation. The thickness and material in the strips 17,18 is chosen in such a manner that a visual change, a whitening, is obtained when the tension in the side portion reaches a value between the lowest value for ensuring a good fit of the article and the value causing risk for damage of the skin, preferably well beyond the lower limit, if possible. The value chosen is preferably lower than the value causing risk for skin irritation or damage.

The strips 17,18 are attached to the respective side portion of the rear part 5 by any suitable means, such as glue or weld joins. In the disclosed embodiment the strips are attached to casing sheet 2 along their whole length but it is feasible to attach the strip to casing sheet 2 only at their opposed lateral ends.

When the article according to the embodiment shown in Figures 1-3 is applied to a wearer, the side portions of the front and rear parts are first attached to each other with an overlap on one side of the article, in the example shown in Figure 2, the right side of the article when viewed from the front by attaching the tab 14 to the strip 16. Since the waistline of the article remains open after this measure, no appreciable tensioning of the waistline is obtained. It is first when the side portions of the front and rear parts opposite to the already attached side portions, are attached to each other with the help of fastener elements 13 and 15 that the waistline must be tensioned in order to attain a tight fit of the article. The person applying the article in the way indicated in Figure 2 grips the tab 13 or the side portion to the left in Figure 2 and pulls this portion in over the corresponding side portion of the front part of the article in an overlapping relationship. The greater overlap, the higher tension in the side portions. When the tension indicator 17 whitens, an appropriate overlap has been reached and the tape tab 13 is then attached to the plastic strip 15. By the provision of the tension indicator 17 it is thus very easy for a user to determine when an appropriate tension is applied.

From the foregoing description it is clear that it is enough to provide one of the side portions with a tension indicator. However, if only indicator 18 was present on the article according to Figure 2 and the article was applied in the way described above, the person applying the article would have to study the indicator 18 on the opposite side portion to the portion last applied, when determining the appropriate overlap. This might be inconvenient and for this reason it is preferred that both of the side portions of the rear part are provided with a tension indicator.

Instead of separate strips 17,18, the tabs 13,14 or the side portions of the rear part 5 can be constructed to serve as tension indicators.

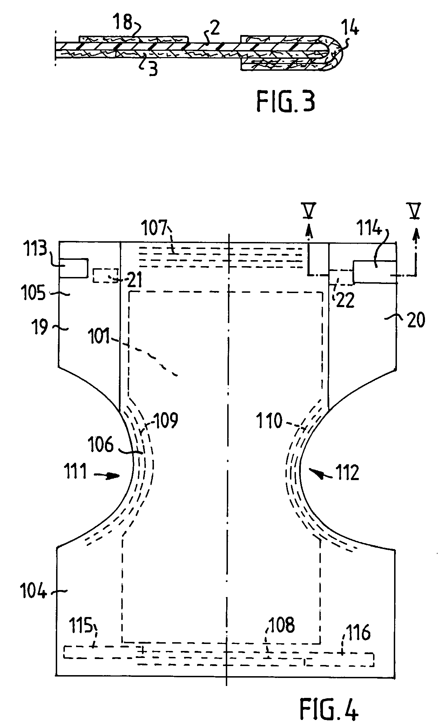

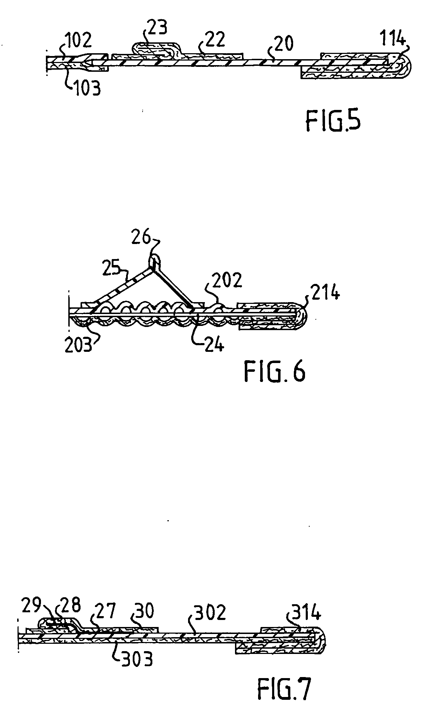

In Figure 4 and 5, an article according to a second embodiment of the invention is shown in views corresponding to Figure1 and 3, respectively. The article according to this embodiment differs from the article according to Figures 1-3 in that the side portions of the rear part are of elastic material, such as elastic nonwoven or the like, and constitute side panels 19, 20 attached to the casing sheets 102,103. The components of the article according to the second embodiment similar to corresponding components of the article according to the first embodiment shown in Figures 1-3 are given the same reference numerals in Figures 4 and 5 with the addition of 100. The reason for providing the article with elastic side panels 19,20 is to improve comfort and the overall fit of the article. When such an article is applied to a wearer the side panels will be elongated in a lateral direction. The materials used for tension indicators can normally not follow the elongation desired for the side panels without rupture. The tension indicators 21, 22 according to the second embodiment are therefore attached to the outer side of the side panels 19,20 in a folded condition with only the lateral ends thereof attached to the panel, as can be seen in Figure 5. This means that in the start of the lateral elongation of the panels, no tension will be introduced in the tension indicators, the elongation of the panel 20 leading to an out-folding of the fold 23 of tension indicator 22. The lateral length of the fold 23 may be chosen so that the lowest value for ensuring a good fit of the article is reached in the side panels when the tension indicator 22 is totally unfolded. The tension at which the visual appearance of the tension will occur may then be chosen near the value causing risk for damage of the skin. In the second embodiment according to Figures 4 and 5 the tension indicators 21,22 are a combination of two different tension indicators, one in which the unfolding of the tension indicator visually tells the reader that a certain tension is reached, and the other being the colour change in the material, for example whitening due to cold flow deformation.

Instead of providing separate side panels of elastic material, the side portions of the rear part can be elasticised by providing pre-tensioned elastic threads between the casing sheets, said threads being attached to one or both of the casing sheets. Such side portions are present in a third embodiment of an article according to the invention, a side portion of such an article is shown in Figure 6 in a view similar to Figure 3, after the pre-tensioned threads has been able to relax after manufacture. The components of the article according to the third embodiment similar to corresponding components of the article according to the first embodiment shown in Figures 1-3 are given the same reference numerals in Figure 6 with the addition of 200. When the means holding the elastic threads in a pre-tensioned state during manufacture are removed, said threads strive to resume their initial length, i.e. to shorten and thereby recover to a tension free state. The recovery of the elastic threads lead to a gathering of the casing sheets 202, 203 attached thereto, as is evident from Figure 6. An elongate tension indicator 25 attached in its lateral ends to the outer casing sheet 202 during manufacture, i.e. with the elastic threads in a pre-tensioned state, will also be gathered so that a fold occurs between its ends. In order to make use of the elasticity of the elastic threads in the side portions, some gathering of the casing sheets 202,203 shall remain in the applied condition of the article. In order for the tension indicator 25 to be subjected to tension when some gathering of the casing sheets remains, the tension indicator must be shortened after its gathering due to the recovery of the elastic threads. In the embodiment shown in Figure 6, such a shortening is accomplished by affixing parts of the sides facing each other in the fold, for example by a glue or weld join 26 as is schematically indicated in Figure 6. Thereafter, the indicator may be folded to a neat configuration similar to the fold 23 of the second embodiment disclosed in Figure 5.

A fourth embodiment of an article according to the invention is shown in Figure 7. The components of the article according to the fourth embodiment similar to corresponding components of the article according to the first embodiment shown in Figures 1-3 are given the same reference numerals in Figure 7 with the addition of 300. The only difference between the first and fourth embodiment is the construction of the tension indicator. The tension indicator 27 of the fourth embodiment is releasable and refastenable in relation to the casing sheet 302, for example by being attached thereto by a suitable adhesive. The attachment is however strong enough to resist the maximum tension the tension indicator will be subjected to. Furthermore, the tension indicator 27 contains a fold 28, the sides of the indicator facing each other in the fold being attached to each other by a join 29 being of the same type as the join attaching the tension indicator to casing sheet 302. The tension indicator 27 may also have an end portion 30 unattached to casing sheet 302 in order to facilitate release of the tension indicator. By such a construction it is possible to readjust an article after a first adjustment leading to a first visual change of the indicator, the first visual change occurring in end part of the indicator turned against the fastener 314. When a readjustment is to be done, the user grips the free end 30, unfolds fold 28 and attach the unfolded indicator to casing sheet 302 in a stretched state, the unfolded part of the fold constituting a new indicating section of the tension indicator 27. In a variant the end of the indicator opposite to end 30 might be more firmly attached to casing sheet 302 than the rest of the indicator 27. It is of course possible to provide the tension indicator with more than one fold 28 if more than possibility of readjustment is needed.

The visual change of the material can of course be other than the appearance of a colour or the unfolding of a fold. For example, an opaque indicator can turn transparent when a certain tension therein is reached. It is also possible to use rupture of a material as a visual indication. In such a case it would be advantageous to foresee that the indicator has another colour than the underlying material by colouring the indicator or the underlying material. The indicator can be a laminate of a strip of rupturing material and a strip of elastic material having a different colour than the rupturing material. Such a laminate construction can also be used for the other types of indicators mentioned above.

An overly or unsufficiently tight fit of absorbent articles of the above mentioned types can also result from applying articles of a too small or a too large size for the wearer in question. It is therefore appropriate to provide tension indicators on said articles in order to indicate if an appropriate size of the article is chosen. Such indicators can be placed on other parts of the article, for example adjacent to the leg elastics. For a pant diaper a tension indicator can suitably be provided in one or both of the hip regions of the diaper. Preferably, tension indicators for indicating too low tension as well as too high tension are provided. It is also possible to construct the whole backsheet from a material that changes its visual appearance when the tension in the material is overly high. It is advantageous to also provide such a backsheet with other types of tension indicators indicating if sufficient tension is obtained in sensible regions of the backsheet, such as in the leg elastics and in the side portions of the article.

The described embodiments can of course be modified within the scope of the invention. For example, the article can be a diaper for children instead of a diaper for adults. It is also possible to provide indicators parts of the article outside the waist region. The tension indicator need not be separate from the casing sheets but a weakened portion of an outer casing sheet can be used as tension indicator. More than one indicator, each indicator indicating a different tension in the waist region, can be used. Furthermore, the article can have different configuration than disclosed. For example can the absorbent body have a rectangular shape and the article can lack waist elastics. The invention shall therefore only be limited by the content of the enclosed patent claims.

## Claims

1. A disposable absorbent article having a front part (4; 104), a rear part (5; 105) and a crotch part (6;106) extending between the front and rear part, wherein the article comprises fasteners (13,14; 113,114;214;314) for fastening the side portions of the front and rear parts together in an overlapping relationship in order to give the absorbent article a pants-like configuration, the overlap between the side portions of the respective front and rear part being adjustable, the longitudinal direction of the article runs from the rear to the front part, wherein at least one of the side portions of the front or rear parts (4,5; 104,105) comprises at least one tension indicator (17,18;21,22;25;27) **characterised in that** the tension indicator (17,18;21,22;25;27) consists of a strip of plastic material that change its visual appearance when a certain tension is applied thereto, and wherein said plastic material is transparent until a certain tension is reached and then becomes opaque or said plastic material is opaque until a certain tension is reached and then becomes transparent.

2. The article according to Claim 1, **characterised in that** the article comprises elastic elements (9,10) along leg openings (11,12) and that at least one tension indicator is disposed adjacent to at least one of said leg openings.

3. The article according to Claim 1, **characterised in that** a tension indicator (21,22;25) is disposed visually on the outside of the article in at least one side part thereof and is placed in a region between a free end of said front or rear part and a leg opening.

4. The article according to Claim 1, **characterised in that** an underlying colour appears when said plastic material becomes transparent.

5. The article according to any one of Claims 1-4, **characterised in that** the article comprises tension indicators of two different types, wherewith one of said indicator types indicate that a minimum tension level is reached and the other that a maximum tension level is reached.

6. The article according to any one of Claims 1 or 4-5, **characterised in that** said strip (17,18;21,22;25;27) of plastic material is attached to an outside of the article (4,5; 104,105) only in the opposite lateral ends of the strip.

7. The article according to Claim 6, **characterised in that** said strip (22;25;27) of plastic material comprises a fold (23;28) between its lateral ends.

8. The article according to Claim 1, **characterised in that** at least one side portion of the rear or front part of the article comprises an outer sheet of a material which visual appearance changes when a certain tension is reached in the material.

## Patentansprüche

1. Saugfähiger Einwegartikel mit einem Vorderteil (4;104), einem Rückteil (5;105) und einem Schrittteil (6;106), das sich zwischen dem Vorder- und dem Rückteil erstreckt, wobei der Artikel Verschlussmittel (13,14;113,114;214;314) umfasst, um die Seitenabschnitte des Vorder- und Rückteils in überlappender Beziehung aneinander zu befestigen, um dem saugfähigen Artikel eine hosenartige Konfiguration zu verleihen, wobei die Überlappung der Seitenabschnitte des Vorder- bzw. Rückteils einstellbar ist, wobei die Längsrichtung des Artikels vom Rück- zum Vordereilteil verläuft, wobei zumindest einer der Seitenabschnitte des Vorder- oder Rückteils (4,5;104,105) zumindest einen Spannungsindikator (17,18;21,22;25;27) umfasst, **dadurch gekennzeichnet, dass** der Spannungsindikator (17,18;21,22;25;27) aus einem Streifen aus Kunststoffmaterial besteht, der sein visuelles Erscheinungsbild verändert, wenn eine gewisse Spannung an ihn angelegt wird, und wobei das Kunststoffmaterial transparent ist, bis eine gewisse Spannung erreicht ist, und dann opak wird, oder das Kunststoffmaterial opak ist, bis eine gewisse Spannung erreicht ist, und dann transparent wird.

2. Artikel nach Anspruch 1, **dadurch gekennzeichnet, dass** der Artikel Beinöffnungen (11,12) entlang elastische Elemente (9,10) umfasst, und dass zumindest ein Spannungsindikator angrenzend an zumindest eine der Beinöffnungen angeordnet ist.

3. Artikel nach Anspruch 1, **dadurch gekennzeichnet, dass** ein Spannungsindikator (21,22;25) visuell an der Außenseite des Artikels in zumindest einem Seitenteil davon angeordnet und in einem Bereich zwischen einem freien Ende des Vorder- oder Rückteils und einer Beinöffnung platziert ist.

4. Artikel nach Anspruch 1, **dadurch gekennzeichnet, dass** eine darunterliegende Farbe erscheint, wenn das Kunststoffmaterial transparent wird.

5. Artikel nach einem der Ansprüche 1-4, **dadurch gekennzeichnet, dass** der Artikel zwei unterschiedliche Arten von Spannungsindikatoren umfasst, wobei eine der Indikatorarten anzeigt, dass ein Mindestwert der Spannung erreicht ist, und der andere, dass ein Höchstwert der Spannung erreicht ist.

6. Artikel nach einem der Ansprüche 1 oder 4-5,**dadurch gekennzeichnet, dass** der Streifen (17,18;21,22;25;27) aus Kunststoffmaterial nur an den gegenüberliegenden seitlichen Enden des Streifens an einer Außenseite des Artikels (4,5;104,105) angebracht ist.

7. Artikel nach Anspruch 6 **dadurch gekennzeichnet, dass** der Streifen (22;25;27) aus Kunststoffmaterial zwischen seinen seitlichen Enden eine Falte (23; 28) umfasst.

8. Artikel nach Anspruch 1, **dadurch gekennzeichnet, dass** zumindest ein Seitenabschnitt des Rück- oder Vorderteils des Artikels eine äußere Lage aus einem Material umfasst, dessen visuelles Erscheinungsbild sich verändert, wenn eine gewisse Spannung im Material erreicht ist.

## Revendications

1. Article absorbant jetable ayant une partie avant (4 ; 104), une partie arrière (5 ; 105) et une partie d'entrejambe (6 ; 106) s'étendant entre la partie avant et la partie arrière, l'article comprenant des éléments de fixation (13, 14 ;113, 114 ; 214 ; 314) pour fixer les parties latérales des parties avant et arrière l'une à l'autre dans une relation de chevauchement afin de donner à l'article absorbant une configuration de type culotte, le chevauchement entre les parties latérales de la partie avant et arrière respective étant ajustable, la direction longitudinale de l'article va de la partie arrière vers la partie avant, dans lequel au moins une des parties latérales des parties avant ou arrière (4, 5 ;104, 105) comprend au moins un indicateur de tension (17, 18 ; 21, 22 ; 25 ; 27), **caractérisé en ce que** l'indicateur de tension (17, 18 ; 21, 22 ; 25 ; 27) est composé d'une bande de matériau en plastique qui modifie son apparence visuelle lorsqu'une certaine tension y est appliquée, et dans lequel ledit matériau en plastique est transparent jusqu'à ce qu'une certaine tension soit atteinte et il devient ensuite opaque ou ledit matériau en plastique est opaque jusqu'à ce qu'une certaine tension soit atteinte et il devient ensuite transparent.

2. Article selon la revendication 1, **caractérisé en ce que** l'article comprend des éléments élastiques (9,10) le long des ouvertures de jambes (11,12) et **en ce qu'**au moins un indicateur de la tension est placé de façon adjacente à l'au moins une desdites ouvertures de jambe.

3. Article selon la revendication 1, **caractérisé en ce que** l'indicateur de tension (21,22;25) est placé visuellement sur l'extérieur de l'article dans au moins une partie latérale de celui-ci et est placé dans une région entre une extrémité libre de ladite partie avant ou arrière et une ouverture de jambe.

4. Article selon la revendication 1, **caractérisé en ce qu**'une couleur sous-jacente apparaît lorsque ledit matériau en plastique devient transparent.

5. Article selon l'une quelconque des revendications 1 à 4, **caractérisé en ce que** l'article comprend des indicateurs de tension de deux types différents, avec lequel l'un desdits types d'indicateurs indique qu'un niveau minimal de tension est atteint et l'autre indique qu'un niveau de tension maximal est atteint.

6. Article selon l'une quelconque des revendications 1 ou 4 à 5, **caractérisé en ce que** ladite bande (17, 18 ; 21, 22 ; 25 ; 27) de matériau en plastique est fixée à un extérieur de l'article (4, 5 ; 104, 105) seulement dans les extrémités latérales opposées de la bande.

7. Article selon la revendication 6, **caractérisé en ce que** ladite bande (22 ; 25 ; 27) de matériau en plastique comprend un pli (23 ; 28) entre ses extrémités latérales.

8. Article selon la revendication 1, **caractérisé en ce qu**'au moins une partie latérale de la partie avant ou arrière de l'article comprend une feuille externe d'un matériau dont l'apparence visuelle change lorsqu'une certaine tension est atteinte dans le matériau.
